# EUROPÄISCHE PATENTSCHRIFT

(11) **EP 0 350 925 B1**
(45) Veröffentlichungstag und Bekanntmachung des Hinweises auf die Patenterteilung: **12.10.1994**
(21) Anmeldenummer: 89112861.3
(22) Anmeldetag: 13.07.1989
(51) Int. Cl.: A61B 17/60

(54) **Externe Knochenbruchteil-Verbindungsvorrichtung**
External clamping device for a bone fracture
Dispositif d'immobilisation externe pour fracture osseuse

(30) Priorität: 13.07.1988 DE 3823746
(43) Veröffentlichungstag der Anmeldung: 17.01.1990
(73) Patentinhaber: KARL LEIBINGER MEDIZINTECHNIK GMBH & CO., D-78570 Mühlheim (DE)
(72) Erfinder: Bohrmann, Peter, Ing. (grad.), D-7202 Mühlheim (DE); Ballier, Roland, Dr. med., D-7205 Böttingen (DE)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) Entgegenhaltungen:
- WO-A-88/01152
- CH-A- 323 411
- FR-A- 789 882
- GB-A- 2 033 758
- US-A- 3 096 110

## Beschreibung

Die Erfindung betrifft eine externe Knochenbruchteil-Verbindungsvorrichtung zur mechanischen Verbindung der Bruchteile eines gebrochenen Knochens, insbesondere des gebrochenen Unterschenkels, nach dem Oberbegriff des Patentanspruchs 1.

Bei einer bekannten derartigen externen Knochenbruchteil-Verbindungsvorrichtung (WO-A-88/01152) sind für die Fixierung der Knochenhaltestangen als auch der Verbindungsstangen jeweils Klemmbackenpaare vorgesehen, die zur Aufnahme der Knochenhaltestangen bzw. der Verbindungsstangen entsprechend geformte, gegenüberliegende Nuten aufweisen, welche sich bei einer die Klemmbackenpaare unter einer vorbestimmten Winkelstellung gegeneinander festlegenden Verspannung der Klemmbackenpaare fest an die Knochenhaltestangen bzw. die Verbindungsstangen anlegen, so daß diese gegeneinander und gegenüber den Klemmbackenpaaren fixiert sind.

Die Drehglieder der bekannten Knochenbruchteil-Verbindungsvorrichtung sind im Vergleich zur Ausdehnung der zu verbindenden Stangen sehr voluminös ausgebildet und empfindlich gegen bei der Montage dort eindringende Fremdkörper, welche die definierte Klemmung der Knochenhaltestangen behindern oder gar verhindern können. Darüber hinaus weisen die Klemmbackenpaare verhältnismäßig große und zum Teil schwer zugängliche Anlageflächen auf, wodurch die hygienische Aufbereitung der Klemmbackenpaare vor ihrem Einsatz erschwert wird.

Das Ziel der vorliegenden Erfindung besteht darin, eine externe Knochenbruchteil-Verbindungsvorrichtung der eingangs genannten Gattung zu schaffen, die ein insbesondere auch noch nachträgliches Anbringen oder Entfernen von Drehgliedern sowohl an den Verbindungsstangen als auch den Knochenhaltestangen ermöglicht und gleichzeitig eine einfache Handhabbarkeit auch insbesondere unter Berücksichtigung der besonderen hygienischen Erfordernisse bei einem klinischen Einsatz aufweist.

Zur Lösung dieser Aufgabe sind die Merkmale des kennzeichnenden Teils des Patentanspruchs 1 vorgesehen.

Aufgrund dieser Ausbildung erstrecken sich die Nuten radial so weit nach außen, daß man bei der Herstellung der Drehglieder mit einem Minimum an Material auskommt, ohne daß deren Festigkeit beeinträchtigt wird. Da praktisch der gesamte Innenraum der Drehglieder nur von den Knochenhaltestangen und Verbindungsstangen ausgefüllt ist, können sich nirgends Fremdkörper festsetzen und die Funktion der Vorrichtung beeinträchtigen. Gleichzeitig wird hierdurch eine einfache Sauberhaltung bzw. Aufbereitbarkeit der Vorrichtung gewährleistet.

Dynamische Verbindungsvorrichtungen, bei denen eine Verschiebbarkeit der Gelenke verwirklicht ist, sind in der Zeitschrift "Akt. Traumatol. 17 (1987)" auf den Seiten 86 bis 90 beschrieben. Bei derartigen dynamisierten Verbindungsvorrichtungen erstrecken sich die Knochenhaltestangen vorzugsweise senkrecht zur Knochenachse und liegen in ein und derselben Ebene. Zwei parallel zueinander angeordnete Verbindungsstangen verlaufen im wesentlichen parallel zur Knochenachse und sind ebenfalls vorzugsweise in einer gemeinsamen Ebene angeordnet. Die Gelenke zwischen den Knochenhaltestangen und den Verbindungsstangen sind so ausgebildet, daß die eine Verbindungsstange jeweils in dem Gelenk des einen Knochenbruchteils und die andere Verbindungsstange in dem Gelenk des anderen Knochenbruchteils axial geführt gleiten kann. Hierdurch werden die beiden Bruchteile einerseits in exakter axialer Ausrichtung gehalten, während andererseits in Achsrichtung eine Verschiebung möglich ist. Dies hat den Sinn, bei der Belastung des Bruches eine gewisse definierte Nachgiebigkeit der Verbindungsvorrichtung zu gewährleisten, wodurch der Knochenheilungsprozeß begünstigt wird.

Diese bekannte Verbindungsvorrichtung hat den Nachteil, daß die axiale Relativverschiebung der beiden Verbindungsstangen nur durch zusätzlich vorgesehene Anschläge begrenzt werden kann.

Die Handhabbarkeit der erfindungsgemäßen externen Knochenbruch-Verbindungsvorrichtung der eingangs genannten Art wird in dieser Hinsicht zusätzlich durch die Merkmale des Patentanspruchs 6 günstig beeinflußt.

Durch die spezielle Ausbildung einer Gleitnut an einer der Klemmbacken wird eine definierte Verschiebbarkeit der Gelenke relativ zueinander längs der Verbindungsachse ermöglicht, ohne daß es hierfür eines Lösens der gegenseitigen Verspannung der Klemmbacken bedarf, da ein Lösen der Verspannung der Klemmbacken zum Verschieben der Klemmbacken bei dem erfindungsgemäßen Gelenk nicht notwendig ist, besteht auch nicht die Gefahr, daß sich bei der Manipulation des Gelenkes die Winkelausrichtung der Klemmbacken gegeneinander verstellt.

Zur Verwirklichung einer dynamischen Verbindungsvorrichtung können sowohl die erfindungsgemäßen Verbindungsstangen-Klemmbacken, die ein streng axial geführtes Gleiten einer der Verbindungsstangen durch das Gelenk zulassen, als auch gattungsgemäß ausgebildete Verbindungsstangen-Klemmbacken vorgesehen sein, die eine kraftschlüssige Verklemmung beider Verbindungsstangen am Gelenk ermöglichen. Der Orthopäde oder Chirurg kann auf diese Weise wahlweise eine vollständige Fixierung oder eine im vorstehenden Sinne dynamisierte Fixierung der Bruchteile eines Knochens herbeiführen, wobei sich die Anbringungsmöglichkeit für die einzelnen Drehglieder an beliebigen Stellen der Verbindungsstangen besonders vorteilhaft auswirkt, denn es ist auf diese Weise beispielsweise möglich, eine beide Verbindungsstangen am Gelenk festklemmende Verbindungsstangen-Klemmbacke an Ort und Stelle problemlos durch eine erfindungsgemäße Klemmbacke zu ersetzen, wenn dies beispielsweise im Verlauf des Knochenheilungsprozesses als vorteilhaft angesehen werden sollte. Dem Arzt stehen somit zwei verschiedene und einfach anzuwendende, miteinander kombinierbare Möglichkeiten für die Fixierung eines Unterschenkelbruches zur Verfügung.

Die vorliegende Erfindung schafft ein System, bei dem einfach ein Verbindungsstangen-Klemmbackenpaar als Anschlag für ein erfindungsgemäßes Gelenk verwendet werden kann, ohne daß - mit Ausnahme eines kürzeren Verbindungsbolzens - irgendwelche Anpassungsmaßnahmen erforderlich sind. Insbesondere können zusätzliche Drehglieder an beliebigen Stellen der Knochenhaltestangen als auch der Verbindungsstangen insbesondere auch noch nachträglich einzeln oder in gewünschter Weise miteinander kombiniert angebracht werden, ohne daß zuvor schon festgeklemmte Drehglieder entfernt werden müssen.

Vorteilhafte Weiterbildungen in der Erfindung ergeben sich aus den weiteren Unteransprüchen.

Die Erfindung wird im folgenden beispielsweise anhand der Zeichnung beschrieben; in dieser zeigt:
- Fig. 1: einen axialen Querschnitt eines bei der erfindungsgemäßen Verbindungsvorrichtung vorgesehenen Gelenks,
- Fig. 2: eine Draufsicht des Gelenks nach Fig. 1 in Richtung des Pfeiles II in Fig. 1,
- Fig. 3: eine teilweise aufgebrochene Draufsicht des Gelenkes nach Fig. 1 in Richtung des Pfeiles III in Fig. 1,
- Fig. 4: eine Draufsicht einer an einem Knochenbruch angeordneten erfindungsgemäßen Verbindungsvorrichtung bei einseitig offener im wesentlichen ebener Rahmenanordnung,
- Fig. 5: eine Ansicht des Gegenstandes der Fig. 4 in Richtung des Pfeiles V in Fig. 4,
- Fig. 6: eine zu Fig. 4 ähnliche Ansicht mit einer anderen Anordnung der Anschläge,
- Fig. 7: eine Ansicht des Gegenstandes der Fig. 6 in Richtung des Pfeiles VII in Fig. 6,
- Fig. 8: eine zu den Fig. 4 und 6 ähnliche Ansicht mit einer weiteren möglichen Anordnung der Anschläge,
- Fig. 9: eine Ansicht des Gegenstandes der Fig. 8 in Richtung des Pfeiles IX in Fig. 8,
- Fig. 10: eine ähnliche Ausschnittsansicht wie die Fig. 5, 7 und 9, wobei jedoch zusätzlich noch eine Distanzscheibe vorgesehen ist und
- Fig. 11: eine schematische perspektivische Ansicht einer dreidimensionalen, zeltartigen Verbindungsvorrichtung gemäß der Erfindung.

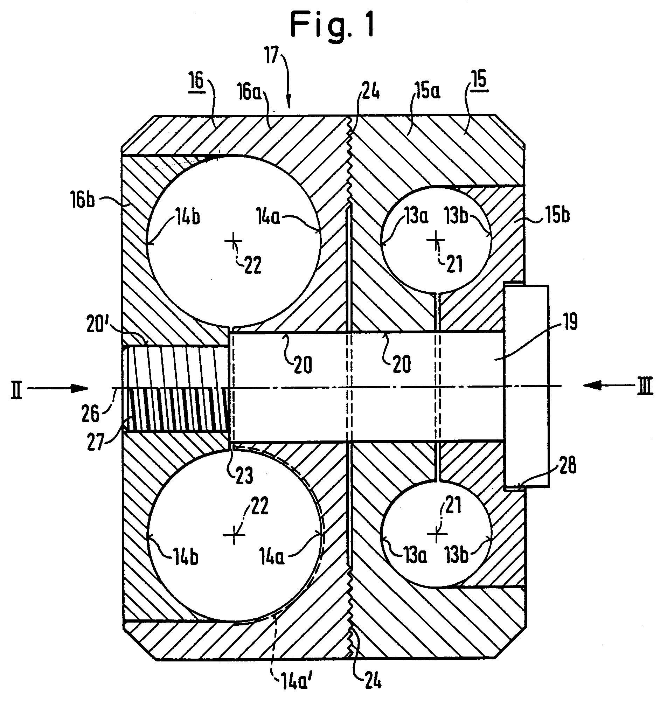

Nach den Fig. 1 bis 3 bestehen die Gelenke 17 einer erfindungsgemäßen externen Knochenbruchteil-Verbindungsvorrichtung jeweils aus zwei im wesentlichen kreiszylindrischen und miteinander kongruenten Drehgliedern 15, 16, die an ihren einander zugewandten Stirnflächen jeweils gleich ausgebildete Radialverzahnungen 24 aufweisen, welche konzentrisch zur Mittelachse 26 der Drehglieder 15, 16 verlaufen.

In der Mitte der in etwa scheibenförmig ausgebildeten Drehglieder 15, 16 befinden sich zentrale Verbindungsbohrungen 20 bzw. 20′, wobei die Bohrung 20′ einen etwas geringeren Durchmesser als die Verbindungsbohrungen 20 aufweist und mit einem Innengewinde 27 versehen ist. Von der freien Stirnseite des Drehgliedes 15 ist ein mit einem Kopf versehener Verbindungsbolzen 19 durch die Verbindungsbohrungen 20 hindurchgesteckt und in die am gegenüberliegenden Ende vorgesehene mit Innengewinde 27 versehene Bohrung 20′ eingeschraubt, wobei sich der Kopf des Verbindungsbolzens 19 in eine dazu komplementäre Vertiefung 28 des Drehgliedes 15 setzt und so die beiden Drehglieder 15, 16 axial miteinander verspannt und auch axial ausrichtet.

Bei etwas gelöstem Verbindungsbolzen 19 können die beiden Drehglieder 15, 16 um die Mittelachse 26 verdreht werden, wobei die Rastverzahnungen 24 jeweils von einem Zahn zum nächsten springen. Durch erneutes Festziehen des Verbindungsbolzens 19 kann die einmal eingestellte Winkellage fixiert werden.

Erfindungsgemäß bestehen die Drehglieder 15, 16 jeweils aus zwei Klemmbacken 15a, 15b bzw. 16a, 16b, von denen die aneinanderliegenden Klemmbacken 15a bzw. 16a, die mit den Radialverzahnungen 24 versehen sind, ein in der Ansicht der Fig. 1 im wesentlichen U-förmiges Basisteil bilden, welches auf der von der Radialverzahnung 24 abgewandten Stirnfläche mit im wesentlichen halbkreiszylindrisch ausgebildeten Nuten 13a bzw. 14a versehen sind.

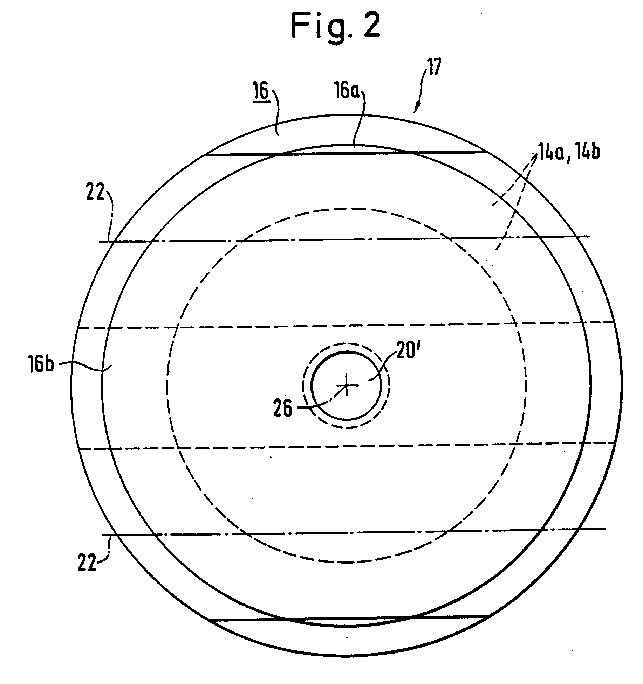

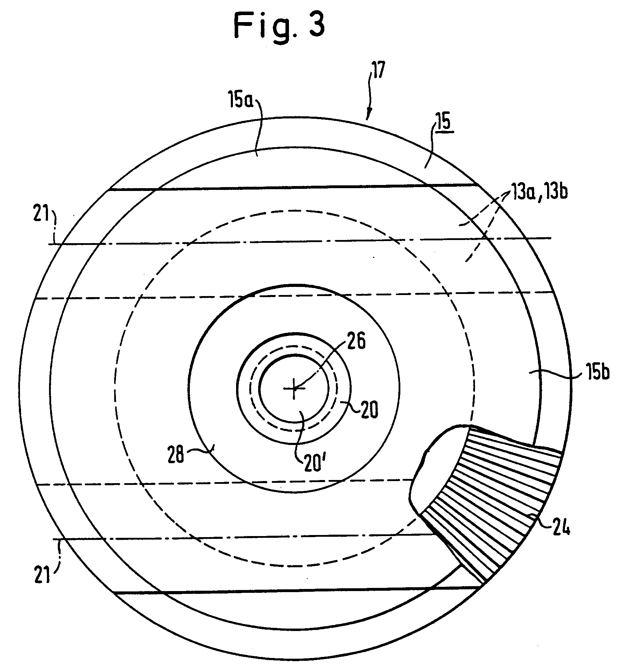

In die voneinander abgewandten Öffnungen der mit einem im wesentlichen U-förmigen Querschnitt versehenen, als Basisteile ausgebildeten Klemmbacken 15a bzw. 15b sind Klemmteile bildende zweite Klemmbacken 15b bzw. 16b eingesetzt, welche die aus den Fig. 2 und 3 ersichtliche abgeflachte Kreisform aufweisen. Die Klemmbacken 15b, 16b weisen im wesentlichen ebene äußere Stirnflächen auf und sind axial gegenüber den Nuten 13a, 14a mit ebenfalls annähernd halbkreiszylindrisch ausgebildeten und parallel zu den Nuten 13a, 14a verlaufenden Nuten 13b bzw. 14b versehen, wodurch insgesamt die aus Fig. 1 ersichtlichen kreiszylindrischen Klemmkanäle mit den Längsachsen 21 bzw. 22 gebildet werden. Die Verbindungsbohrungen 20, 20′ sind durch entsprechende Bohrungen in den Klemmbacken 15a, 15b, 16a, 16b verwirklicht, wobei die Verbindungsbohrungen 20 in den Klemmbacken 15a, 15b, 16a und die Gewindebohrung 20′ in der letzten Klemmbacke 16b vorgesehen sind.

Der untere linke kreiszylindrische Klemmkanal mit der Längsachse 22 kann auch mit einer etwas größeren Gleitnut 14a′ in der Klemmbacke 16a versehen sein, deren Sinn und Zweck weiter unten im einzelnen beschrieben wird.

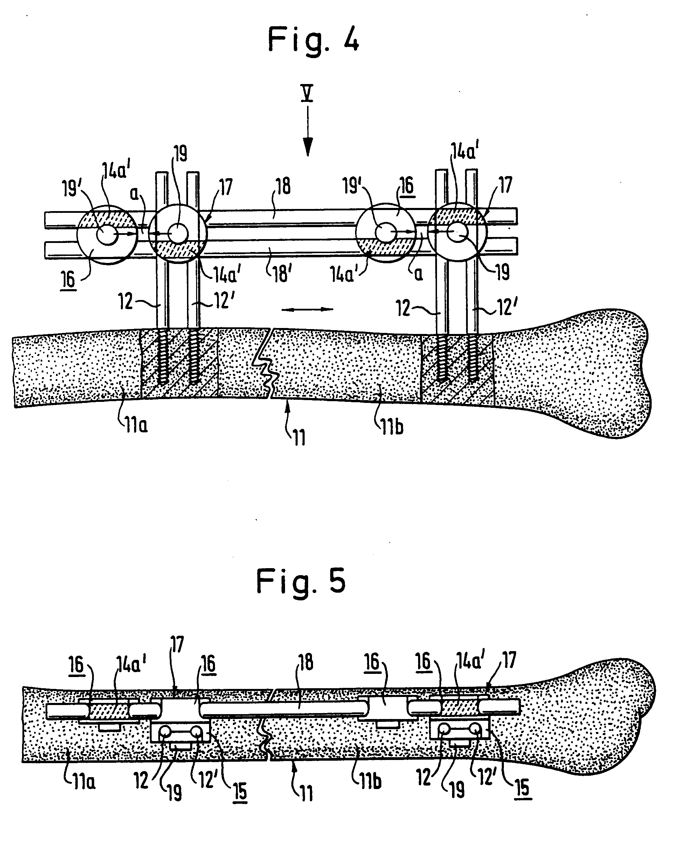

Die durch die Nuten 13a, 13b gebildeten kreiszylindrischen Klemmkanäle, welche ebenso wie die durch die Nuten 14a, 14b gebildeten Klemmkanäle mit ihrer Längsachse 21 senkrecht zur Mittelachse 26 der Drehglieder 15, 16 verlaufen, dienen nach den Fig. 4 und 5 zur Aufnahme und Klemmung von zwei parallel zueinander und in einer Ebene angeordneten Knochenhaltestangen 12, 12′, die in die beiden Bruchteile 11a, 11b eines Knochens 11 eingeschraubt sind, und zwar im wesentlichen senkrecht zur Längsachse des Knochens 11.

Die durch die Nuten 14a, 14b gebildeten kreiszylindrischen Klemmkanäle mit den Längsachsen 22 weisen einen deutlich größeren Durchmesser als die durch die Nuten 13a, 13b gebildeten Klemmkanäle auf und dienen zur im wesentlichen formschlüssigen Aufnahme von zwei parallel zueinander angeordneten entsprechend kreiszylinderförmigen Verbindungsstangen 18, 18′ (Fig. 4, 5). Durch Anziehen des Verbindungsbolzens 19 (Fig. 1, 4, 5) kann die Position der Knochenhaltestange 12, 12′ und der Verbindungsstangen 18, 18′ gemäß den Fig. 4, 5 festgelegt werden.

Wird die Ausführungsform mit der etwas größeren Gleitnut 14a′ (Fig. 1) verwendet, so ist es erforderlich, daß die beiden Klemmbacken 16a, 16b sich im Bereich der Gleitnut 14a′ nicht nur auf den Verbindungsstangen 18, 18′, sondern auch unmittelbar an einer Verbindungsstelle 23 aneinander abstützen, und zwar derart, daß die Verbindungsstange 18 oder 18′ an der betreffenden Stelle zwar formschlüssig und axial geführt gehalten, nicht jedoch axial unverschieblich festgeklemmt wird.

In den Fig. 4 und 5 ist die axialverschiebliche Lagerung der Verbindungsstangen 18 bzw. 18′ in den Drehgliedern 16 durch eine Rauhschattierung und die Bezugszahl 14a′ angedeutet. Nach Fig. 4 ist also bei dem rechten Gelenk 17 die obere Verbindungsstange 18, beim linken Gelenk 17 die untere Verbindungsstange 18′ axial verschiebbar im zugeordneten Drehteil 16 gelagert, während die jeweils andere Verbindungsstange in dem betreffenden Gelenk 17 vollständig festgeklemmt ist. Auf diese Weise können die beiden Gelenke 17 in Richtung des Doppelpfeiles in Fig. 4 relativ zueinander verschoben werden. Um die axiale Verschiebung der beiden Gelenke 17 nach den Fig. 4 und 5 in Richtung des Doppelpfeiles zu begrenzen, sind links neben den Gelenken 17 separate Drehglieder 16 mittels verkürzter Verbindungsbolzen 19′ auf den Verbindungsstangen 18, 18′ angeordnet, wobei eine definierter geringer Abstand a zu den Drehgliedern 16 der Gelenke 17 eingestellt ist. Die Summe der beiden Abstände a ergibt den Gesamtlängsverschiebungsbereich der Gelenke 17 in Richtung des Doppelpfeiles relativ zueinander.

Damit die zusätzlichen Drehglieder 16 als Anschläge wirken können, müssen dort die Gleitnuten 14a′ gerade in umgekehrter Anordnung wie bei den zugeordneten gelenken 17 vorgesehen werden.

Die Anordnung der Gewindebohrung 20′ (Fig. 1) in der äußeren Klemmbacke 16b des Verbindungsstangen-Drehgliedes 16 hat also auch den Zweck, die separate Benutzung der Drehglieder 16 als Anschläge im Sinne von Fig. 4, 5 mit verkürzten Verbindungsbolzen 19′ zu ermöglichen.

Die Handhabung der erfindungsgemäßen Verbindungsanordnung ist wie folgt:
Zunächst werden die Knochenhaltestangen 12, 12′ gemäß den Fig. 4, 5 möglichst senkrecht zur Achse des Knochens 11 in die Bruchteile 11a, 11b eingeschraubt. Alsdann werden die Gelenke 17 mit den Verbindungsstangen 18, 18′ angebracht, und die gewünschte Relativlage der beiden Bruchteile 11a, 11b wird dann durch Beobachtung eines Röntgenbildes der Bruchstelle eingestellt und durch Festziehen der Verbindungsbolzen 19 fixiert.

Soll eine Dynamisierung erreicht werden, ist es auch möglich, daß an den Gelenken 17 von vornherein Klemmbacken 16b mit gleitnut 14a′ in der aus Fig. 4 und 5 ersichtlichen Anordnung vorgesehen werden, wobei dann die am Beginn des Heilungsprozesses zweckmäßige vollständige Fixierung des Bruches auch dadurch verwirklicht werden kann, daß der Abstand a in Fig. 4 durch entsprechende Verschiebung der als Anschläge wirkenden Drehglieder 16 zu Null gemacht wird.

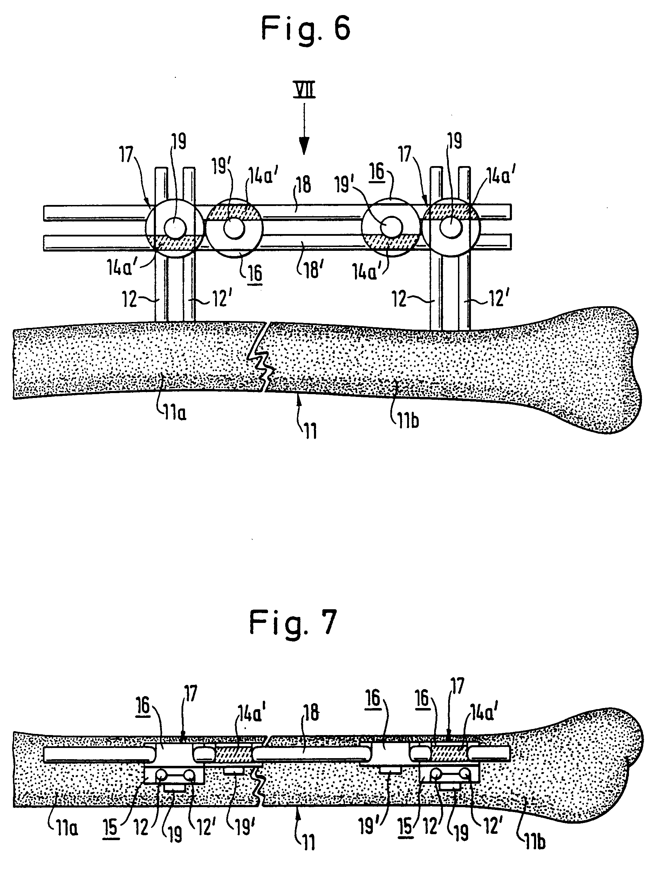

Eine derartige Ausbildung ist in den Fig. 6 und 7 dargestellt, wobei das in Fig. 4 linke, als Anschlag wirkende separate Drehglied 16 jedoch rechts von dem Gelenk 17 angebracht ist, was ebenfalls möglich ist.

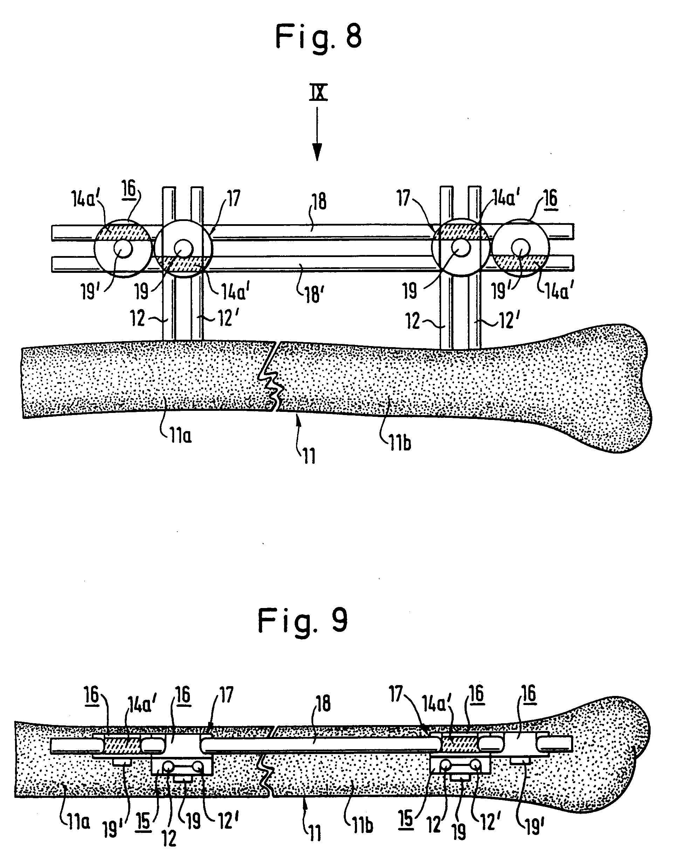

Die Fig. 8 und 9 zeigen eine andere mögliche Anordnung der als Anschläge wirkenden separaten Drehglieder gegenüber Fig. 4, wobei nunmehr das rechte, als Anschlag wirkende Drehglied 16 rechts vom Gelenk 17 angeordnet ist. Der Abstand a ist auch bei dem Ausführungsbeispiel nach den Fig. 8 und 9 zu Null gemacht worden, kann jedoch durch problemlose axiale Verschiebung der separaten Drehglieder 16 vom Arzt jederzeit wiederhergestellt werden.

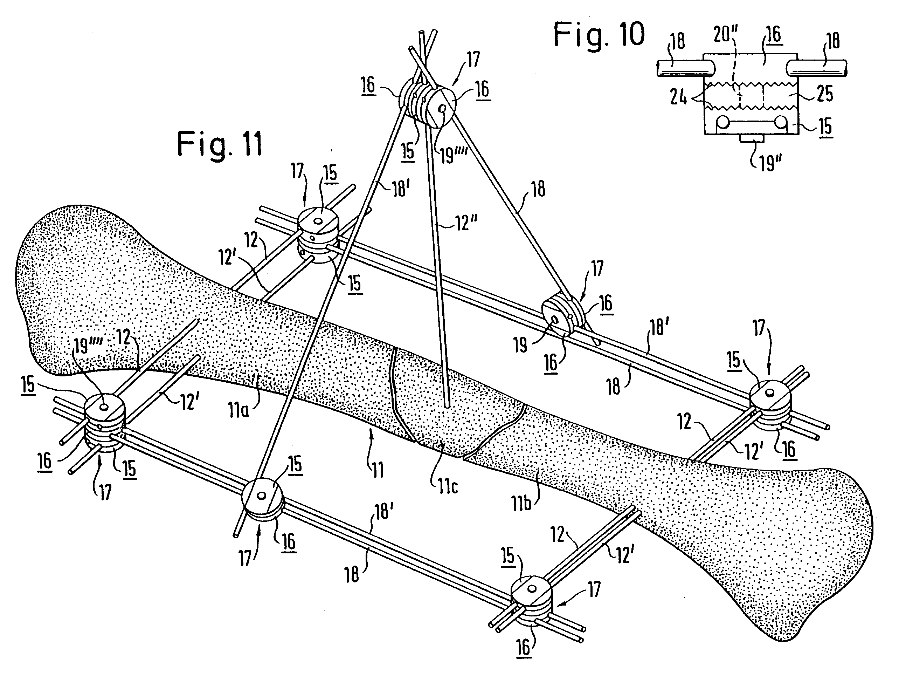

Fig. 10 zeigt, daß zwischen zwei Drehgliedern 15, 16 auch noch eine entsprechend dimensionierte Distanzscheibe 25 angeordnet werden kann, welche zweckmäßigerweise an beiden Stirnseiten die Radialverzahnungen 24 aufweist, mittels deren sie bei Verspannung durch den Verbindungsbolzen 19˝ die beiden Drehglieder 15, 16 drehfest miteinander verspannen kann. Die Distanzscheiben 25 können in unterschiedlichen Dicken vorrätig gehalten werden, wobei der Verbindungsbolzen 19˝ entsprechend länger als beim Ausführungsbeispiel nach den Fig. 1 bis 3 auszubilden ist.

Durch entsprechend mit unterschiedlicher Länge ausgebildete Verbindungsbolzen und in verschiedener Weise miteinander kombinierte Drehglieder 15, 16 bzw. Distanzscheiben 25 können auch räumlich kompliziertere Verbindungsvorrichtungen verwirklicht werden, wobei ein Beispiel in Fig. 11 perspektivisch dargestellt ist.

Die Knochenstangen 12, 12′ durchgreifen zum Teil in verschiedenen Höhe die Bruchteile 11a, 11b des Knochens 11 vollständig und münden auf entgegengesetzten Seiten des Knochens 11 jeweils in Drehgliedern 15, wo sie erfindungsgemäß festgeklemmt sind. Die einzelnen Drehglieder 15 auf den entgegengesetzten Seiten des Knochens 11 sind mittels der Drehglieder 16 und der Verbindungsstangen 18, 18′ miteinander verbunden, wobei ggfs. eine Dynamisierung gemäß den Fig. 4, 5 vorgesehen sein könnte.

Da die in Fig. 11 linken Knochenhaltestangen 12, 12′ in der Höhe versetzt sind, sind auf beiden Seiten eines mittleren Drehgliedes 16 zwei Knochenstangenhalte-Drehglieder 15 angeordnet, wozu ein entsprechend lang ausgebildeter Verbindungsbolzen 19˝˝ zu verwenden ist. Entsprechend ist die Ausbildung des gegenüberliegenden Gelenks 17.

Der Knochen 11 weist bei dem Ausführungsbeispiel nach Fig. 11 in der Mitte ein herausgebrochenes Stück 11c auf, welches durch eine zeltstangenartig von oben eingebohrte Knochenhaltestange 12˝ relativ zu den Bruchteilen 11a, 11b fixiert werden kann, wobei die Halterung der Knochenhaltestange 12˝ durch zeltartig aufgestellte Verbindungsstangen 18, 18′ erfolgt, die über zwei beidseits eines mittleren Drehgliedes 15 vorgesehene Verbindungsstangen-Drehglieder 16 in der dargestellten Position fixiert werden. Die Verbindung mit dem in der Ebene des Knochens 11 befindlichen Rahmen erfolgt über jeweils zwei miteinander zusammengebaute Verbindungsstangen-Drehglieder 16.

Aus dem Ausführungsbeispiel nach Fig. 11 ergibt sich, daß die Verbindungsstangen-Drehglieder 16 nicht nur gemäß den Fig. 4 bis 9 allein als Anschläge, sondern auch paarweise zur Verbindung von Verbindungsstangen unter verschiedenen Winkeln verwendet werden können. Nimmt man noch die Distanzscheiben 25 gemäß Fig. 10 als weiteres Bauelement hinzu, so können mit der erfindungsgemäßen Verbindungsanordnung auch komplizierte räumliche Verbindungsvorrichtungen in gewünschter Weise verwirklicht werden.

Die beiden Klemmbacken 16a, 16b der Verbindungsstangen-Drehglieder 16 sind zwar bevorzugt gemäß Fig. 1 als ineinandergeschachtelte Bauteile ausgebildet, doch könnten grundsätzlich auch zwei vollständig zur Berührungsebene zueinander symmetrische Klemmbacken verwendet werden.

## Patentansprüche

1. Externe Knochenbruchteil-Verbindungsvorrichtung zur mechanischen Verbindung der Bruchteile (11a, 11c) eines gebrochenen Knochens (11), insbesondere des gebrochenen Unterschenkels, mit quer in die Bruchteile (11a, 11b, 11c) eingeführten Knochenhaltestangen (12, 12', 12''), welche außerhalb des Knochens (11) und des ihn enthaltenden Gliedes zwischen zwei mit zu den Knochenhaltestangen (12, 12', 12'') komplementären, gegenüberliegenden Nuten (13a, 13b) versehene Klemmbacken (15a, 15b), welche auseinandergenommen werden können, klemmbar sind, welche gemeinsam das erste Drehglied (15) eines Gelenkes (17) bilden, dessen zweites Drehglied (16) durch einen Verbindungsbolzen (19) gegenüber dem ersten Drehglied (15) in verschiedenen Winkelpositionen festlegbar und mit Verbindungsstangen (18, 18') zur mechanischen Verbindung mehrerer Gelenke (17) versehen ist, wobei das zweite Drehglied (16) aus zwei mit zu den Verbindungsstangen (18, 18') komplementären Nuten (14a, 14b) versehenen Klemmbacken (16a, 16b) besteht und wobei die Klemmbacken (15a, 15b; 16a, 16b) aus einem im Querschnitt U-förmigen Basisteil (15a, 16a) und einem dazu komplementären, in die Öffnung des U vorzugsweise formschlüssig einsetzbaren Klemmteil (15b, 16b) bestehen, wobei die Nuten (13a, 14a; 13b, 14b) sich am Boden des U bzw. der gegenüberliegenden Fläche des Klemmteils (15b, 16b) befinden,
dadurch **gekennzeichnet,**
daß die Nuten 13a, 14a; 13b, 14b) der Klemmbacken (15a, 15b; 16a, 16b) sich bis zum radial äußeren Rand des Innenraumes der im Querschnitt U-förmigen Basisteile (15a, 16a) erstrecken.

2. Vorrichtung nach Anspruch 1,
dadurch **gekennzeichnet,**
daß die Nuten (13a, 14a) der Basisteile (15a, 16a) radial außen stetig in eine zylindrische Wand übergehen, deren Achse mit der Mittelachse (26) der Drehglieder (15, 16) zusammenfällt.

3. Vorrichtung nach Anspruch 1 oder 2,
dadurch **gekennzeichnet,**
daß der Verbindungsbolzen (19) bzw. die für ihn vorgesehenen Bohrungen (20, 20') senkrecht zu den Längsachsen (21, 22) der Nuten (13a, 13b; 14a, 14b) verlaufen.

4. Vorrichtung nach einem der vorhergehenden Ansprüche,
dadurch **gekennzeichnet,**
daß der Verbindungsbolzen (19) durch eine Verbindungsbohrung (20) in den Klemmbacken (15a, 15b; 16a, 16b) in eine Gewindebohrung (20') der vom Kopf des Verbindungsbolzens (19) aus gesehen letzten Klemmbacke (16b) eingeschraubt ist.

5. Vorrichtung nach einem der vorhergehenden Ansprüche,
dadurch **gekennzeichnet,**
daß die Verbindungsstangen-Klemmbacken (16a, 16b) und auch die Knochenhaltestangen-Klemmbacken (15a, 15b) jeweils zwei beidseitig der Verbindungsbohrung (20, 20') vorgesehene Nutenpaare (13a, 13b; 14a; 14b) aufweisen.

6. Vorrichtung nach einem der vorhergehenden Ansprüche,
dadurch **gekennzeichnet,**
daß die Verbindungsstangen-Klemmbacken (16a, 16b) und die Knochenhaltestangen-Klemmbacken (15a, 15b) jeweils zwei beidseitig der Verbindungsbohrung (20, 20') vorgesehene Nutenpaare (13a, 13b; 14a, 14b) aufweisen und daß wenigstens eine Klemmbacke (16a) der Verbindungsstangen-Klemmbackenpaare (16a, 16b) eine derart vergrößerte Gleitnut (14a') aufweist und sich neben der betreffenden Nut (14a') auch unmittelbar (23) derart an der anderen Klemmbacke (16b) abstützt, daß die in diese Gleitnut (14a') und die entsprechende Gegennut (14b) eingesetzte Verbindungsstange (18, 18') bei festgeschraubtem Verbindungsbolzen (19) axial geführt in dem Drehglied (16) verschiebbar ist.

7. Vorrichtung nach Anspruch 6,
dadurch **gekennzeichnet,**
daß nur eine der Klemmbacken (16a) der Verbindungsstangen-Klemmbackenpaare (16a, 16b) in der beschriebenen Weise ausgebildet ist.

8. Vorrichtung nach Anspruch 6 oder 7,
dadurch **gekennzeichnet,**
daß als Anschläge neben den Gelenken (17) reine Verbindungsstangen-Klemmbackenpaare (16a, 16b) anbringbar sind, welche wenigstens eine Gleitnut (14a') an derjenigen Verbindungsstange (18') aufweisen, mit der das zugeordnete Gelenk (17) fest verklemmt ist.

9. Vorrichtung nach einem der vorhergehenden Ansprüche,
dadurch **gekennzeichnet,**
daß die Drehglieder (15, 16) auf den aneinanderliegenden Flächen eine zur Verbindungsbohrung (20, 20') konzentrische und miteinander in Eingriff stehende Radialverzahnung (24) aufweisen.

10. Vorrichtung nach einem der vorhergehenden Ansprüche,
dadurch **gekennzeichnet,**
daß Distanzscheiben (25) vorgesehen sind, die eine Verbindungsbohrung (20'') für den Verbindungsbolzen (19) aufweisen und an beiden Stirnflächen komplementär zu den einander zugewandten Flächen der Drehglieder (15, 16) ausgebildet sind.

11. Vorrichtung nach einem der vorhergehenden Ansprüche,
dadurch **gekennzeichnet,**
daß die Drehglieder (15, 16) kreiszylindrisch ausgebildet sind.

12. Vorrichtung nach Anspruch 10,
dadurch **gekennzeichnet,**
daß die Distanzscheiben (25) kreiszylindrisch ausgebildet sind.

13. Vorichtung nach Anspruch 11,
dadurch **gekennzeichnet,**
daß die Durchmesser der Drehglieder (15, 16) gleich groß sind.

14. Vorrichtung nach Anspruch 11 bis 13,
dadurch **gekennzeichnet,**
daß die Durchmesser der Drehglieder (15; 16) und der Distanzscheibe (25) gleich groß sind.

15. Vorrichtung nach einem der vorhergehenden Ansprüche,
dadurch **gekennzeichnet,**
daß die Nutenpaare (13a, 13b; 14a, 14b) eines Drehgliedes (15; 16) parallel zueinander angeordnet sind.

16. Vorrichtung nach einem der vorhergehenden Ansprüche,
dadurch **gekennzeichnet,**
daß die Nutenpaare (13a, 13b; 14a, 14b) eines Drehgliedes (15, 16) im gleichen Abstand von der Mittelachse (26) der Verbindungsbohrungen (16, 16') angeordnet sind.

## Claims

1. External fixation apparatus for fractured bone parts for the mechanical connection of the fractured parts (11a, 11c) of a broken bone (11), in particular of the broken lower leg-with bone holding bars (12, 12', 12'') which are introduced transversely into the fractured parts (11a, 11b, 11c) and which can be clamped outside of the bone (11) and of the limb containing it between two clamping jaws (15a, 15b) provided with oppositely disposed grooves (13a, 13b) complementary to the bone holding bars (12, 12', 12'') which can be separated from one another and which jointly form the first rotational member (15) of a hinge (17), the second rotational member (16) of which can be fixed via a connecting bolt (19) in various angular positions relative to the first rotary member (15) and is provided with connecting bars (18, 18') for the mechanical connection of a plurality of hinges (17), with the second rotary member (16) consisting of two clamping jaws (16a, 16b) with grooves (14a, 14b) complementary to the connecting bars (18, 18'), and with the clamping jaws (15a, 15b; 16a, 16) consisting of a base part (15a, 16a) which is U-shaped in cross-section and a clamping part 15b, 16b) complementary thereto which can be inserted in preferably form-locked manner into the opening of the U, with the grooves (13a, 14a; 13b, 14b) being located at the base of the U, or of the oppositely disposed surface of the clamping part (15b, 16b),
**characterised in that**
the grooves (13a, 14a;13b, 14b) of the clamping jaws (15a, 15b; 16a, 16b) extend up to the radially outer edge of the inner space of the base part (15a, 16a) of U-shaped cross-section.

2. Apparatus in accordance with claim 1,
**characterised in that**
the grooves (13a, 14a) of the base parts (15a, 16a) merge radially outwardly continuously into a cylindrical wall, the axis of which coincides with the central axis (26) of the rotary members (15, 16).

3. Apparatus in accordance with claim 1 or 2,
**characterised in that**
the connection bolt (19) or the bore (20, 20') provided for it extend perpendicularly to the longitudinal axes (21, 22) of the grooves (13a, 13b; 14a, 14b).

4. Apparatus in accordance with one of the preceding claims, **characterised in that**
the connection bolt (19) is screwed through a connecting bore (20) in the clamping jaws (15a, 15b; 16a, 16b) into a threaded bore (20') of the last clamping jaw (16b) as seen from the head of the connecting bolt (19).

5. Apparatus in accordance with one of the preceding claims, **characterised in that**
the connecting bar clamping jaws (16a, 16b) and also the bone holding bar clamping jaws (15a, 15b) each have two groove pairs (13a, 13b; 14a, 14b) provided on both sides of the connecting bore (20, 20').

6. Apparatus in accordance with one of the preceding claims, **characterised in that**
the connecting bar clamping jaws (16a, 16b) and the bone holding bar clamping jaws (15a, 15b) respectively have two groove pairs (13a, 13b; 14a, 14b) provided on both sides of the connecting bore (20, 20'); in that at least one clamping jaw (16a) of the connecting bar clamping jaw pairs (16a, 16b) has an enlarged slide groove (14a') and is also supported alongside the relevant groove (14a') directly (23) against the other clamping jaw (16b) such that the connecting bar (18, 18') inserted into this slide groove (14a') and into the corresponding counter-groove (14b) is axially displaceably guided in the rotary member (16) with the connecting bolt (19) screwed tight.

7. Apparatus in accordance with claim 6,
**characterised in that**
only one of the clamping jaws (16a) of the connecting bar clamping jaw pairs (16a, 16b) is formed in the described manner.

8. Apparatus in accordance with claim 6 or claim 7,
**characterised in that**
pure connecting bar clamping jaw pairs (16a, 16b) can be placed adjacent the joints (17) as abutments and have at least one slide groove (14a') at that connecting bar (18'), with which the associated hinge (17) is fixedly clamped.

9. Apparatus in accordance with one of the preceding claims, **characterised in that**
the rotary members (15, 16) have radial tooth arrangements (24) on the contacting surfaces which are concentric to the connection bore (20, 20') and engage with one another.

10. Apparatus in accordance with one of the preceding claims, **characterised in that**
spacer discs (25) are provided which have a connection bore (20'') for the connecting bolt (19) and are formed at both end faces complementary to the confronting faces of the rotary members (15, 16).

11. Apparatus in accordance with one of the preceding claims, **characterised in that**
the rotary members (15, 16) are of right cylindrical shape.

12. Apparatus in accordance with claim 10,
**characterised in that**
the spacer discs (25) are of right cylindrical shape.

13. Apparatus in accordance with claim 11,
**characterised in that**
the diameters of the rotary members (15, 16) are of the same size.

14. Apparatus in accordance with claims 11 to 14,
**characterised in that**
the diameters of the rotary members (15; 16) and of the spacer discs (25) are of the same size.

15. Apparatus in accordance with one of the preceding claims, **characterised in that**
the groove pairs (13a, 13b; 14a, 14b) of a rotary member (15; 16) are arranged parallel to one another.

16. Apparatus in accordance with one of the preceding claims, **characterised in that**
the groove pairs (13a, 13b; 14a, 14b) of a rotary member(15; 16) are arranged at the same distance from the central axis (26) of the connection bores (16, 16').

## Revendications

1. Appareil de liaison pour des fragments osseux afin de relier mécaniquement les fragments (11a, 11c) d'un os (11) brisé, en particulier d'un tibia brisé, comprenant des tiges de maintien (12, 12', 12'') introduites transversalement dans les fragments osseux (11a, 11b, 11c), ces tiges pouvant être serrées à l'extérieur de l'os (11) et du membre qui contient cet os entre deux mâchoires de serrage (15a, 15b) qui comportent des gorges opposées (13a, 13b) complémentaires des tiges de maintien (12, 12', 12''), ces mâchoires de serrage pouvant démontées l'une par rapport à l'autre, et lesdites mâchoires formant ensemble le premier organe rotatif (15) d'une articulation (17) dont le second organe rotatif (16) peut être fixé au moyen d'un boulon de raccordement (19) dans différentes positions angulaires par rapport au premier organe rotatif (15), et ledit second organe rotatif étant pourvu de tiges de liaison (18, 18') pour la liaison mécanique de plusieurs articulations (17), le second organe rotatif (16) étant constitué par deux mâchoires de serrage (16a, 16b) pourvues de gorges (14a, 14b) complémentaires des tiges de liaison (18, 18'), les mâchoires de serrage (15a, 15b ; 16a, 16b) étant constituées d'une pièce de base (15a, 16a) ayant en coupe transversale la forme d'un U, et d'une pièce des serrage (15b, 16b) complémentaire à la pièce de base et pouvant être introduite dans l'ouverture du U, de préférence en verrouillage de forme, les gorges (13a, 14a ; 13b, 14b) étant situées au fond du U, ou de la surface en vis-à-vis de la pièce de serrage (15b, 16b),
caractérisé en ce que les gorges (13a, 14a ; 13b, 14b) des mâchoires de serrage (15a, 15b ; 16a, 16b) s'étendent jusqu'à la bordure radiale extérieure de la cavité intérieure des pièces de base (15a, 16a) ayant en coupe transversale la forme d'un U.

2. Appareil selon la revendication 1, caractérisé en ce que les gorges (13a, 14a) des pièces de base (15a, 16a) se transforment radialement à l'extérieur progressivement en une paroi cylindrique dont l'axe coïncide avec l'axe médian (26) des organes rotatifs (15, 16).

3. Appareil selon l'une ou l'autre des revendications 1 et 2, caractérisé en ce que le boulon de liaison (19), ou respectivement les perçages (20, 20') prévus pour celui-ci, s'étendent perpendiculairement aux axes longitudinaux (21, 22) des gorges (13a, 13b ; 14a, 14b).

4. Appareil selon l'une quelconque des revendications précédentes, caractérisé en ce que le boulon de liaison (19) est vissé à travers un perçage de liaison (20) dans les mâchoires de serrage (15a, 15b ; 16a, 16b) dans un perçage taraudé (20') de la dernière mâchoire de liaison (16b) vue depuis la tête du boulon de liaison (19).

5. Appareil selon l'une quelconque des revendications précédentes, caractérisé en ce que les mâchoires de serrage (16a, 16b) pour les tiges de liaison, et les mâchoires de serrage (15a, 15b) pour les tiges de maintien de l'os, comportent respectivement deux paires de gorges (13a, 13b ; 14a, 14b) prévues des deux côtés du trou de liaison (20, 20').

6. Appareil selon l'une quelconque des revendications précédentes, caractérisé en ce que les mâchoires de serrage (16a, 16b) pour les tiges de liaison et les mâchoires de serrage (15a, 15b) pour les tiges de maintien comportent respectivement deux paires de gorges (13a, 13b ; 14a, 14b) des deux côtés du trou de liaison (20, 20'), et en ce que l'une au moins des mâchoires de serrage (16a) de la paire de mâchoires de serrage (16a, 16b) pour les tiges de liaison comportent une gorge de coulissement (14a') agrandie, et s'appuie également directement sur l'autre mâchoire de serrage (16b) à côté de la gorge considérée (14a'), de telle manière que les tiges de liaison (18, 18') introduites dans cette gorge de coulissement (14a') et dans la contre-gorge correspondante (14b) peuvent être déplacées en étant guidées axialement dans l'organe rotatif (16) tandis que le boulon de liaison est fermement serré (19).

7. Appareil selon la revendication 6, caractérisé en ce que seule l'une des mâchoires de serrage (16a) de la paire de mâchoires de serrage (16a, 16b) pour les tiges de liaison est réalisée de la manière décrite.

8. Appareil selon l'une ou l'autre des revendications 6 et 7, caractérisé en ce que des paires de mâchoires de serrage (16a, 16b) uniquement pour des tiges de liaison peuvent être montées en tant que butées à côté des articulations (17), ces paires de mâchoires de serrage comportant au moins une gorge de coulissement (14a') au niveau de celles des tiges de liaison (18') avec laquelle l'articulation associée (17) est fermement serrée.

9. Appareil selon l'une quelconque des revendications précédentes, caractérisé en ce que les organes rotatifs (15, 16) comportent sur les surfaces en contact des dentures radiales (24) concentriques aux perçages de liaison (20, 20') et en engrènement l'une avec l'autre.

10. Appareil selon l'une quelconque des revendications précédentes, caractérisé en ce que des disques d'écartement (25) sont prévus, qui comportent un perçage de liaison (20'') pour le boulon de liaison (19) et qui sont réalisés sur leurs deux surfaces frontales de façon complémentaire aux surfaces en vis-à-vis des organes rotatifs (15, 16).

11. Appareil selon l'une quelconque des revendications précédentes, caractérisé en ce que les organes rotatifs (15, 16) sont réalisés sous forme de cylindres à base circulaire.

12. Appareil selon la revendication 10, caractérisé en ce que les disques d'écartement (25) sont réalisés sous la forme de cylindres à base circulaire.

13. Appareil selon la revendication 11, caractérisé en ce que les diamètres des organes rotatifs (15, 16) sont égaux.

14. Appareil selon l'une quelconque des revendications 11 à 13, caractérisé en ce que les diamètres des organes rotatifs (15, 16) et des disques d'écartement (25) sont égaux.

15. Appareil selon l'une quelconque des revendications précédentes, caractérisé en ce que les paires gorges (13a, 13b ; 14a, 14b) d'un organe rotatif (15, 16) sont agencées parallèlement les unes aux autres.

16. Appareil selon l'une quelconque des revendications précédentes, caractérisé en ce que les paires de gorges (13a, 13b ; 14a, 14b) d'un organe rotatif (15, 16) sont agencées à la même distance de l'axe médian (26) des perçages de liaison (16, 16').
